# NEUE EUROPÄISCHE PATENTSCHRIFT

(11) **EP 1 862 784 B2**
(45) Veröffentlichungstag und Bekanntmachung des Hinweises auf die Entscheidung über den Einspruch: **18.11.2015**
(45) Hinweis auf die Patenterteilung: 26.08.2009
(21) Anmeldenummer: 07005135.4
(22) Anmeldetag: 14.03.2007
(51) Int. Cl.: G01G 19/44, A61B 5/053

(54) **Vorrichtung zum Wiegen**
Device for weighing
Dispositif destiné au pesage

(30) Priorität: 02.06.2006 DE 102006026281
(43) Veröffentlichungstag der Anmeldung: 05.12.2007
(73) Patentinhaber: Medisana AG, 41468 Neuss (DE)
(72) Erfinder: Lindner, Ralf, 20149 Hamburg (DE)
(74) Vertreter: Ostriga Sonnet Wirths & Vorwerk

(56) Entgegenhaltungen:
- EP-A- 1 586 267
- EP-A1- 1 091 334
- EP-B1- 1 519 164
- WO-A1-02/100230
- DE-A1- 1 799 004
- DE-A1- 10 347 424
- DE-T2- 60 110 282
- DE-U1- 20 107 336
- DE-U1- 20 311 556

## Beschreibung

Die Erfindung betrifft eine Vorrichtung zum Wiegen, insbesondere von Menschen, mit einem Lastaufnahmebereich, mit der auf mechanische und/oder elektronische Weise vorzugsweise das Gewicht, der Körperfett-, der Wasser-, der Muskel- und/oder der Knochenanteil eines Menschen ermittelt wird, dass der Lastäufnahmebereich aus einer transluzenten Platte gebildet wird, an der bodenseitig Fußelemente angeordnet sind, und dass die Vorrichtung ein Gehäuse aufweist, in dem die Steuerungs- und Anzeigeeinrichtungen sowie mindestens ein Leuchtmittel angeordnet sind, wobei die von dem Leuchtmittel erzeugten Lichtstrahlen zumindest teilweise an der der Lichtquelle gegenüberliegenden Plattenbegrenzung sichtbar austreten

Eine derartige Vorrichtung zum Wiegen ist aus der DE 60110282 T2 bekannt, jedoch wird die Möglichkeit zur Anzeige und Darstellung von Informationen für den Benutzer als verbesserungswürdig angesehen, insbesondere im Hinblick auf die notwendigen Hygienestandards.

Die Aufgabe der Erfindung besteht deshalb darin, eine neue Vorrichtung zum Wiegen, insbesondere von Menschen zu schaffen, die zusätzliche Informationen verbessert darstellt.

Die Lösung der Aufgabe ergibt sich aus den Merkmalen des Anspruchs 1, insbesondere den Merkmalen des Kennzeichenteils, wonach die transluzente Platte sandwichartig aus zwei aneinander befestigten dünnen Platten gebildet wird und wonach die Oberfläche der unteren Platte eingearbeitete Strukturen aufweist, an denen ein Teil der eingeleiteten Lichtstrahlen zur Oberfläche der transluzenten Platte hin reflektiert wird und dort austritt.

Die erfindungsgemäße Vorrichtung zum Wiegen weist den Vorteil auf, dass die äußere Oberfläche keine eingearbeiteten Strukturen aufweist, was rein aus reinigungstechnischen und hygienischen Gründen vorteilhaft ist.

Hierbei kann bei einer Ausführungsform der Erfindung das wenigstens eine Leuchtmittel auch nahe einer Stirnfläche der transluzenten Platte angeordnet sein.

Eine andere Möglichkeit besteht darin, dass wenigstens eine Leuchtmittel innerhalb einer in der transluzenten Platte angeordneten Bohrung zu befestigen. Sofern dieses Leuchtmittel im Winkel von 360 ° Lichtstrahlen in die Plattenebene abgibt, ist somit eine allseitige Beleuchtung der Plattenbegrenzung möglich.

Selbstverständlich ist es auch denkbar, dass mehrere Leuchtmittel nahe einer oder unterschiedlicher Stirnflächen einer transluzenten Platte angeordnet sind, so dass der Effekt der Beleuchtung der Plattenbegrenzung verstärkt wird.

Auch ist eine Variation vorstellbar, bei der die Strahlungsrichtungen, der nahe der Stirnfläche der transluzenten Platte angeordneten Leuchtmittel unterschiedlich sind. Dies hat zum Ziel, gegebenenfalls von einem Standort mehrerer Leuchtmittel aus, eine gleichmäßige Beleuchtung sämtlicher Plattenbegrenzungsbereiche zu schaffen.

Eine besondere Ausführungsform der Erfindung ist zusätzlich dadurch gekennzeichnet, dass die Oberfläche der transluzenten Platte zumindest teilweise eingearbeitete Strukturen aufweist und das in diesen Bereichen ein Teil der eingeleiteten Lichtstrahlen nach oben hin austritt. Bei dieser Ausführungsform kann man neben der Beleuchtung der Plattenbegrenzung zusätzlich beispielsweise auch durch die vorgenannten eingearbeiteten Strukturen, Bereiche kennzeichnen. So ist es denkbar, dass beispielsweise der Trittbereich für die Füße durch eine solche eingearbeitete und beleuchtete Struktur gekennzeichnet ist.

Eine bevorzugte Ausführungsform der Erfindung ist dadurch gekennzeichnet, dass die transluzente Platte aus Glas gebildet ist. In diesem Zusammenhang ist es denkbar, dass die transluzente Platte sandwichartig aus zwei aneinander befestigten dünnen Platten gebildet wird und das die Oberfläche der unteren Platte eingearbeitete Strukturen aufweist, an denen ein Teil der eingeleiteten Lichtstrahlen zur Oberfläche der transluzenten Platte hin reflektiert wird und dort austritt. Bei dieser Ausführungsform wird der Vorteil darin gesehen, dass die äußere Oberfläche keine eingearbeiteten Strukturen aufweist, was rein aus reinigungstechnischen und hygienischen Gründen vorteilhaft ist.

Weitere Vorteile der Erfindung ergeben sich aus den nachfolgenden Unteransprüchen sowie aus der Beschreibung eines Ausführungsbeispiels. Es zeigen:

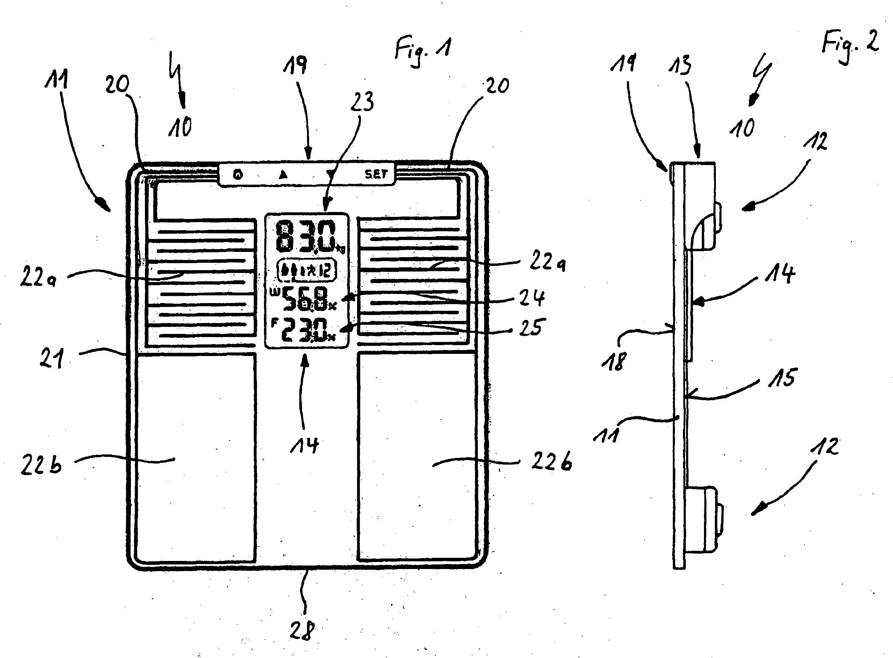

Fig. 1 eine Draufsicht auf eine Vorrichtung zum Wiegen,
Fig. 2 eine Seitenansicht der Vorrichtung gemäß Ansichtspfeil II in Fig. 1,

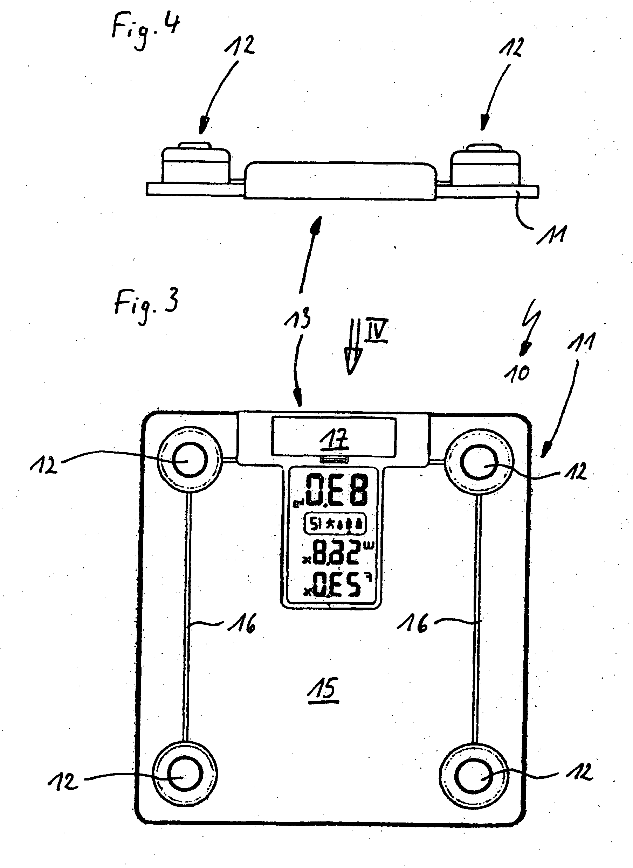

Fig. 3 eine Unteransicht der Vorrichtung gemäß Fig. 1,
Fig. 4 eine Seitenansicht der Vorrichtung gemäß Ansichtspfeil IV in Fig. 3,
Fig. 5 eine Teilansicht bezüglich der Diodenanordnung der Vorrichtung gemäß Fig. 1 und
Fig. 6 eine weitere Teilansicht bezüglich einer anderen Diodenanordnung der Vorrichtung gemäß Fig. 1.

In den Zeichnungen ist eine Vorrichtung zum Wiegen, insbesondere von Menschen insgesamt, mit der Bezugsziffer 10 bezeichnet.

Die Vorrichtung zum Wiegen 10 wird aus einer rechteckigen Glasplatte 11, vier Fußelementen 12, einem Gehäuse 13 und einer Anzeigevorrichtung 14 gebildet. Die Fußelemente 12 sind an einer Unterfläche 15 der Glasplatte 11 befestigt und weisen nicht dargestellte Gewichtssensoren auf. Die von den Gewichtssensoren erfassten Daten werden über auf der Unterseite 15 angeordnete Leiterbahnen 16 dem Gehäuse 13 zugeführt. Das Gehäuse 13 ist ebenfalls im Wesentlichen an der Unterseite 15 der Glasplatte 11 in einem Ausschnitt A letztlich befestigt und weist einerseits auf nicht dargestellte Weise eine Steuerungseinrichtung und andererseits eine an der Unterfläche 15 der Glasplatte angeordnete Anzeigeeinricht ung 14 sowie ein Batteriefach 17 auf.

Auf einer Oberfläche 18 der Glasplatte 11 ist eine Bedienleiste 19 zu erkennen, von der auf der Oberfläche 18 angeordnete Leiterbahnen 20 ausgehen. Diese Leiterbahnen 20 bilden nahe der beiden Längsseiten 21 jeweils unterschiedliche Kontaktflächen 22a und 22b aus, auf die während des Wiegevorgangs die Füße auf nicht dargestellte Weise gestellt werden müssen. Während die Kontaktfläche 22b vollständig geschlossen ausgebildet ist, weist die Kontaktfläche 22a dargestellte Unterbrechung auf. Zwischen den Kontaktflächen 22a ist unterhalb des mittleren Bereiches der Glasplatte 11, die Anzeigevorrichtung 14 zu erkennen. Von oben nach unten erkennt man auf der Anzeigevorrichtung 14 das Gewicht 23 (83 kg), den Wasseranteil 24 (56,8 %) sowie den Fettanteil 25 (23 %).

Das Körpergewicht 23 wird auf einfache Weise über die nicht dargestellten Gewichtssensoren in den Fußelementen 12 gemessen. Dagegen werden der Körperfettanteil und damit auch der Wasseranteil mit Hilfe von den vier Kontaktflächen 22a und 22b erfasst. Bei der Messung muss je ein Fuß gleichzeitig auf einer Kontaktfläche 22a und einer Kontaktfläche 22b stehen. Gemessen wird dann der komplexe Widerstand des Körpers mit einer sogenannten Vierleitermessung. Über zwei Elektroden wird ein kleiner Wechselstrom (< 2 mA) eingeprägt und an den beiden anderen Elektroden die abfallende Spannung gemessen. Der Einfluss des Übergangswiderstandes von den Kontaktflächen zur Haut wird durch das Messverfahren minimiert. Durch das Messen des Widerstandes bei verschiedenen Frequenzen kann anhand eines elektrischen Ersatzmodells des menschlichen Körpers und eines in die Steuerungseinrichtung integrierten Computers der Körperfettanteil errechnet werden.

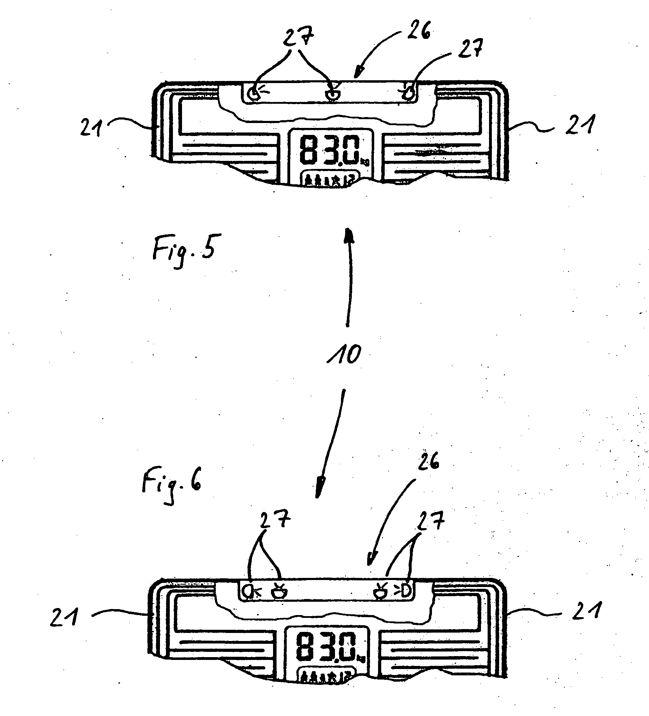

In den Fig. 5 und 6 ist eine besondere Beleuchtung 26 mit einer Mehrzahl von Dioden 27 der Vorrichtung 10 dargestellt. Man erkennt, dass unterhalb der zeichnerisch weggebrochenen Bedienleiste 19 in dem Ausschnitt A der Glasplatte 11 in der Fig. 5 drei Dioden 27 dargestellt sind, bei der eine Diode 27 Lichtstrahlen in die Ebene der Glasplatte 12 in Richtung der gegenüberliegenden Stirnseite 28abstrahlt, während zwei andere Dioden 27 diagonal in Richtung der Längsseiten 21 abstrahlen. Durch diese Art der verdeckten Anordnung der Leuchtdioden 26 unterhalb der Bedienleiste 19 wird es möglich, bei der beschriebenen Ausführungsform lediglich die aus den Längsseiten 21 und den Stirnseiten 28 gebildete Kontur der Vorrichtung 10 auf vorteilhafte Weise zu beleuchten.

Auf nicht dargestellte Weise wäre es grundsätzlich auch möglich, in die Oberfläche der Glasplatte 11 beispielsweise einen Markennamen oder auch gestalterische Strukturen einzuarbeiten, die dann dazu führen würden, dass an dieser Stelle jeweils die hinterleuchte, gut sichtbare Strukturen entstehen würden. So könnte bei einer Körperfettwaage auch die zur Messung notwendige Stellung jedes einzelnen Fußes auf beiden Kontaktflächen 22a und 22b gekennzeichnet werden.

## Patentansprüche

1. Vorrichtung zum Wiegen, insbesondere von Menschen, mit einem Lastaufnahmebereich, in dem auf mechanische und/oder elektronische Weise vorzugsweise das Gewicht, der Körperfett-, der Wasser-, der Muskel- und/oder der Knochenanteil eines Menschen ermittelt wird, dass der Lastaufnahmebereich aus einer transluzenten Platte (11) gebildet wird, an der bodenseitig Fußelemente (12) angeordnet sind und dass die Vorrichtung ein Gehäuse (13) aufweist, in dem Steuerungs- und Anzeigeeinrichtungen (14) sowie mindestens ein Leuchtmittel (27) angeordnet sind, wobei das wenigstens eine Leuchtmittel (27) innerhalb oder außerhalb der transluzenten Platte (11) verdeckt angeordnet ist und wobei die von dem Leuchtmittel (27) erzeugten Lichtstrahlen überwiegend in die Plattenebene eintreten und wobei diese Lichtstrahlen zumindest teilweise an der der Lichtquelle gegenüberliegenden Plattenbegrenzung (21, 28) sichtbar austreten, **dadurch gekennzeichnet, dass** die transluzenten Platte (11) sandwichartig aus zwei aneinander befestigten dünnen Platten gebildet wird und dass die Oberfläche den unteren Platte eingearbeitete Strukturen aufweist, an denen ein Teil der eingeleiteten Lichtstrahlen zur Oberfläche der transluzenten Platte hin reflektiert wird und dort austritt.

2. Vorrichtung nach Anspruch 1, **dadurch gekennzeichnet, dass** das wenigstens eine Leuchtmittel (27) nahe einer Stirnfläche (28) der transluzenten Platte (11) angeordnet ist.

3. Vorrichtung nach Anspruch 1, **dadurch gekennzeichnet, dass** das wenigstens eine Leuchtmittel (27) innerhalb einer in der transluzenten Platte (11) angeordneten Bohrung befestigt ist.

4. Vorrichtung nach einem der vorangehenden Ansprüche, **dadurch gekennzeichnet, dass** mehrere Leuchtmittel (27) nahe einer oder unterschiedlicher Stirnflächen (28) der transluzenten Platte (11) angeordnet sind.

5. Vorrichtung nach einem der vorangehenden Ansprüche, **dadurch gekennzeichnet, dass** die Strahlungsrichtung der nahe der Stirnfläche der transluzenten Platte (11) angeordneten Leuchtmittel unterschiedlich ist.

6. Vorrichtung nach einem der vorangehenden Ansprüche, **dadurch gekennzeichnet, dass** die Oberfläche (18) der transluzenten Platte (11) zumindest teilweise eingearbeitete Strukturen aufweist und dass in diesen Bereichen ein Teil der eingeleiteten Lichtstrahlen nach oben hin austritt.

7. Vorrichtung nach einem der vorangehenden Ansprüche, **dadurch gekennzeichnet, dass** die transluzente Platte (11) aus Glas gebildet ist.

8. Vorrichtung nach einem der vorangehenden Ansprüche, **dadurch gekennzeichnet, dass** als Leuchtmittel Dioden (27) verwendet werden.

9. Vorrichtung nach einem der vorangehenden Ansprüche, **dadurch gekennzeichnet, dass** zur Körpergewichtsmessung mindestens ein in den Fußelementen (12) angeordneter Gewichtssensor vorhanden ist.

10. Vorrichtung nach einem der vorangehenden Ansprüche, **dadurch gekennzeichnet, dass** zur Messung des Körperfettanteils (25) auf der Oberfläche (18) der transluzenten Platte (11) mindestens zwei beabstandete, als Standfläche für die Füße vorgesehene Bereiche vorgesehen sind, die eine dünne ggf. teilweise unterbrochene Kontaktfläche (22a, 22b) aufweisen und dass über die Kontaktflächen (22a, 22b) zum Zwecke der Messung des Körperfettanteils (25) ein geringer Strom durch die auf der Vorrichtung angeordnete Person geleitet wird.

## Claims

1. Device for weighing, in particular of people, with a load bearing area, with which device preferably the weight, the body fat, the water percentage, the muscle mass and/or the bone mass of a person is determined, that the load bearing area is formed from a translucent plate, on the bottom side of which foot elements are arranged, and that the device has a housing, in which the control and display devices as well as at least one lighting means are arranged, and whereas the at least one lighting means (27) is arranged concealed inside or outside the translucent plate (11) and that the light beams produced by the lighting means (27) predominantly enter the plate plane and that these light beams exit visibly at least partly on the plate delimitation (21, 28) lying opposite the light source,
**characterized in that**
the translucent plate (11) is formed in the manner of a sandwich from two thin plates attached to one another and that the surface of the lower plate has incorporated structures, on which a portion of the light beams introduced are reflected towards the surface of the translucent plate and exit there.

2. Device according to claim 1, **characterized in that** the at least one lighting means (27) is arranged close to an end face (28) of the translucent plate (11).

3. Device according to claim 1, **characterized in that** the at least one lighting means (27) is attached inside a hole arranged in the translucent plate (11).

4. Device according to one of the preceding claims, **characterized in that** several lighting means (27) are arranged close to one or different end faces (28) of the translucent plate (11).

5. Device according to one of the preceding claims, **characterized in that** the direction of radiation of the lighting means arranged close to the end face of the translucent plate (11) is different.

6. Device according to one of the preceding claims, **characterized in that** the surface (18) of the translucent plate (11) has at least partly incorporated structures and that in these areas a portion of the light beams introduced exit upwards.

7. Device according to one of the preceding claims, **characterized in that** the translucent plate (11) is formed from glass.

8. Device according to one of the preceding claims, **characterized in that** diodes are used as lighting means (27).

9. Device according to one of the preceding claims, **characterized in that** for measuring body weight at least one weight sensor is present arranged in the foot elements (12).

10. Device according to one of the preceding claims, **characterized in that** to measure body fat (25), at least two areas located at a distance from one another and intended as a standing surface for the feet are provided on the surface (18) of the translucent plate (11), which areas have a thin, if applicable partly interrupted contact surface (22a, 22b) and that via the contact surfaces (22a, 22b) a small current is conducted through the person located on the device for the purpose of measuring body fat (25).

## Revendications

1. Dispositif destiné au pesage, en particulier d'humains, avec une zone support de charge, avec laquelle, de manière mécanique et/ou électronique, de préférence le poids, la teneur en graisses du corps, la teneur en eau, la teneur en muscles et/ou la teneur en os d'un humain sont déterminées, la zone support de charge étant formée d'une plaque translucide, sur laquelle sont disposés des éléments formant pieds, côté sol, et le dispositif présentant un boîtier, dans lequel les dispositifs de commande et d'affichage, ainsi qu'au moins un moyen d'éclairage, sont disposés et que le au moins un moyen d'éclairage (27) est disposé masqué, à l'intérieur ou à l'extérieur de la plaque (11) translucide, et en ce que les rayons lumineux, produits par le moyen d'éclairage (27), pénètrent principalement dans le plan de plaque et en ce que ces rayons lumineux sortent, de façon visible, au moins partiellement, au bord de la plaque (21, 28) opposé à la source lumineuse,
**caractérisé en ce que**
la plaque (11) translucide est formée, à la façon d'un sandwich, de deux plaques minces fixées l'une à l'autre, et **en ce que** la surface de la plaque inférieure présente des structures creusées par usinage, sur lesquelles une partie des rayons lumineux introduits est réfléchie vers la face supérieure de la plaque translucide et sort à cet endroit.

2. Dispositif selon la revendication 1, **caractérisé en ce que** le au moins un moyen d'éclairage (27) est disposé à proximité d'une face frontale (28) de la plaque (11) translucide.

3. Dispositif selon la revendication 1, **caractérisé en ce que** le au moins un moyen d'éclairage (27) est fixé à l'intérieur d'un perçage ménagé dans la plaque (11) translucide.

4. Dispositif selon l'une des revendications précédentes, **caractérisé en ce que** plusieurs moyens d'éclairage (27) sont disposés à proximité d'une ou de différentes faces frontales (28) de la plaque (11) translucide.

5. Dispositif selon l'une des revendications précédentes, **caractérisé en ce que** la direction de rayonnement des moyens d'éclairage, disposés à proximité de la face frontale de la plaque (11) translucide, est différente.

6. Dispositif selon l'une des revendications précédentes, **caractérisé en ce que** la face supérieure (18) de la plaque (11) translucide présente des structures au moins partiellement creusées par usinage et **en ce que** dans ces zones, une partie des rayons lumineux introduits sort vers le haut.

7. Dispositif selon l'une des revendications précédentes, **caractérisé en ce que** la plaque (11) translucide est en verre.

8. Dispositif selon l'une des revendications précédentes, **caractérisé en ce que** l'on utilise des diodes (27) comme moyen d'éclairage.

9. Dispositif selon l'une des revendications précédentes, **caractérisé en ce qu'**au moins un capteur de poids, disposé dans les éléments formant pieds (12), est prévu pour effectuer la mesure du poids corporel.

10. Dispositif selon l'une des revendications précédentes, **caractérisé en ce que**, pour la mesure de la teneur en graisses du corps (25), sur la face supérieure (18) de la plaque (11) translucide sont prévues au moins deux zones espacées, prévues pour faire office de face de placement pour les pieds, présentant une surface de contact (22a, 22b) mince, le cas échéant partiellement interrompue, et **en ce qu'**un faible courant est passé à travers la personne disposée sur le dispositif, par l'intermédiaire des faces de contact (22a, 22b), dans le but de mesurer la teneur en graisses du corps (25).
